# EUROPEAN PATENT SPECIFICATION

(11) **EP 1 758 506 B1**
(45) Date of publication and mention of the grant of the patent: **22.12.2010**
(21) Application number: 05750558.8
(22) Date of filing: 19.05.2005
(51) Int. Cl.: A61B 8/08, A61B 18/12

(54) **3-D ULTRASOUND NAVIGATION DURING RADIO-FREQUENCY ABLATION**
3-D-ULTRASCHALLNAVIGATION WÄHREND HOCHFREQUENZABLATION
NAVIGATION A ULTRASONS 3-D AU COURS D'UNE ABLATION PAR RADIO-FREQUENCE

(30) Priority: 21.05.2004 US 850845
(43) Date of publication of application: 07.03.2007
(73) Proprietor: Boston Scientific Limited, Christ Church (BB)
(72) Inventor: WILLIS, Nathaniel, P., Atherton, CA 94027 (US); CULP, James, M., Los Gatos, CA 95033 (US); SULLIVAN, Vincent, N., San Jose, CA 95125 (US)
(74) Representative: Pfenning, Meinig & Partner GbR
(86) International application number: PCT/US2005/017558
(87) International publication number: WO 2005/112775

(56) References cited:
- WO-A-98/36679
- US-A- 5 840 030
- US-A1- 2003 036 696
- US-A1- 2004 030 249
- US-B1- 6 266 552

## Description

### FIELD OF THE INVENTION

This invention relates to placement of catheters within a body, and more particularly, to navigating and monitoring the positions of catheters utilizing ultrasound during radio-frequency tissue ablation.

### DESCRIPTION OF RELATED ART

The location or position of an instrument or device must be accurately determined before and during various medical procedures and treatments within the body. Common procedures within the body include treating heart conditions, such as supra-ventricular tachycardia (SVT), atrial fibrillation (AF), atrial flutter (AFL) and ventricular tachycardia (VT). SVT, AF, AFT and VT conditions cause abnormal electrical signals to be generated in the endocardial tissue of the heart, which cause irregular beating or arrhythmia of the heart.

Certain heart conditions can be treated by ablating heart tissue using radio frequency (RF) energy. The electrical activity of the heart may be measured using an electrophysiology or "EP" catheter, which includes a mapping electrode to measure the electrical activity.

A map of the electrical activity of the heart can be created and shown on a display. A physician can use the map of EP data to identify which region of the heart is causing irregular activity. An ablation catheter is inserted through the patient's vasculature to the identified target area of the heart. Current produced by a RF generator is applied to target heart tissue to ablate the tissue or form a lesion and treat the heart condition. In other treatments, an ablation catheter is maneuvered into an atrium of the heart to create elongated ablation lesions in the heart and stop irregular beating and other conditions.

Before ablation and EP catheters can be utilized, however, they must be inserted into the body and accurately positioned within the heart. In order to ensure that RF energy is directed to the correct location within the heart, it is usually necessary to monitor or track the position of the catheter before and during ablation.

In conventional systems, catheter monitoring and tracking are typically performed using an imaging system, such as conventional ultrasound imaging or fluoroscopy systems. Once the catheter is positioned, ablation procedures can be performed.

Document US-A-2003/0036696 discloses a system according to the preamble of claim 1. Conventional ultrasound and fluoroscopic imaging systems, particularly when used in conjunction with RF energy, can be improved. For example, some known fluoroscopy systems produce relatively poor quality images that are only in two-dimensions rather than three-dimensions (3-D). These may not produce images having sufficient information and clarity to enable a physician or clinician to effectively perform the procedure. As a further example, known ultrasound imaging systems that continuously monitor the position of a catheter during RF ablation may not be effective since higher-powered RF energy interferes with ultrasound energy. This is particularly problematic when it is necessary to adjust the position of the catheter during RF ablation since the interference may preclude or inhibit a physician or clinician from properly identifying present and future locations of the catheter. Some systems attempt to address this problem by periodically diverting RF energy to another location. Other systems switch between a detection phase of conventional visualization procedures using well known ultrasound imaging equipment and application of RF energy.

These conventional systems, however, may require significant imaging equipment, produce low quality images, and require switching equipment that re-routes RF energy to different locations. Further, conventional ultrasound and fluoroscopy devices are deficient since ablation catheters are routed through the body without establishing the position of the catheter relative to a known reference.

Consequently, even if heart tissue to be ablated can be identified, there may be difficulties with navigating or guiding an ablation catheter to the target or next location within the heart due to an unavailable reference system, low quality ultrasound or fluoroscopic images, and interference caused by high-powered RF ablation energy. Thus, it is desirable to provide a system and method by which a position of a medical device may be accurately and reliably guided to selected regions in the body before and during energy applications.

### SUMMARY

The invention is set out in claim 1. In accordance with one embodiment, a system for positioning a medical device and ablating a tissue within a body include a power supply, a navigation system, and a controller. The power supply generates a current that is suitable for ablating the tissue and provides the current to an energy delivery device. The energy deliver device provides the current to the tissue. The navigation system establishes a three-dimensional reference coordinate system and determines a position of the energy delivery device within the coordinate system. The controller switches between the power supply and navigation system so that the navigation system operates without significant interference from the power supply current during ablation of the tissue.

In accordance with another embodiment, a system for positioning a medical device and ablating a tissue within a body includes a power supply, an energy delivery device, a navigation system, and a controller. The power supply generates current for ablating the tissue and provides the current to the energy delivery device, which provides current to the tissue. The energy deliver device includes isotropic ultrasound transducers. The navigation system includes a reference catheter having a plurality of isotropic ultrasound transducers. The reference catheter transducers emit ultrasound signals to establish a three-dimensional reference coordinate system, and the position of the energy delivery device is determined relative to the coordinate system based on ultrasound signals transmitted and received between the energy delivery device transducer and the reference catheter transducers. The controller switches between activating said power supply and said transducers of the reference catheter and the energy delivery device.

Other embodiments of the gating or switching systems will become apparent from consideration of the following description taken in conjunction with the accompanying drawings.

### BRIEF DESCRIPTION OF THE DRAWINGS

Referring now to the drawings, in which like reference numbers represent corresponding parts throughout, and in which:
Figure 1 illustrates a block diagram of an embodiment of a gating system having a controller in a power supply that switches between ultrasound navigation and RF ablation;
Figure 2 illustrates a block diagram of an embodiment of a gating system having a controller in an ultrasound navigation system that switches between ultrasound navigation and RF ablation;
Figure 3 illustrates timing signals for switching between ultrasound navigation and RF ablation;
Figure 4A illustrates timing signals that include a delay before initiating ultrasound acquisition for switching between ultrasound navigation and RF ablation;
Figure 4B illustrates timing signals that include a delay for use in alternative embodiments;
Figure 5 illustrates a more detailed block diagram of an embodiment of a switching or gating system;
Figure 6 illustrates a more detailed block diagram of an embodiment of switching or gating system having an optional energy trap or RF decay circuit;
Figure 7 is a circuit diagram corresponding to portions of gating logic shown in Figures 5 and 6;
Figure 8 is a circuit diagram illustrating logic and timing components of the gating logic circuit;
Figure 9 illustrates timing signals of the gating logic circuit;
Figure 10 is a circuit diagram illustrating an optical transmitter of the communications interface;
Figure 11 is a circuit diagram corresponding to portions of an energy trap or AF decay circuit;
Figure 12 is circuit diagram illustrating connections between gating system components;
Figure 13 is another circuit diagram illustrating connections between gating system components;
Figure 14 is a schematic representation of major components of an ultrasound navigation system;
Figure 15 is a schematic representation illustrating the manner in which a 3-D reference coordinate system is established;
Figure 16 is a side elevation view of a reference catheter that can be used to establish a 3-D reference coordinate system;
Figure 17 is a side elevation view of another reference catheter;
Figure 18 is a side elevation view of an additional reference catheter;
Figure 19 illustrates an elevation view of a further reference catheter having a different suitable transducer and electrode arrangement; and
Figure 20 illustrates an elevation view of another suitable reference catheter with another suitable transducer and electrode arrangement.

### DETAILED DESCRIPTION OF ILLUSTRATED EMBODIMENTS

Embodiments of a system for gating, alternating or switching between RF ablation of tissue and ultrasound navigation or localization. In the following description, reference is made to the accompanying drawings, which show by way of illustration specific embodiments. It is to be understood that other embodiments may be utilized as various changes to system components and configurations may be made.

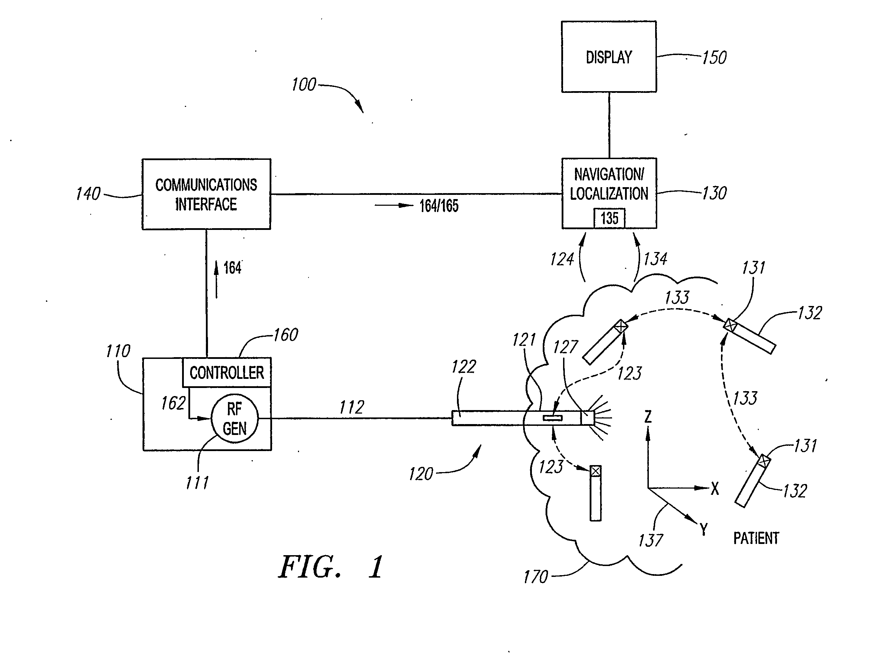

Referring to Figure 1, one embodiment of a gating system 100 for switching between ultrasound navigation or localization and RF ablation of tissue includes a power source or supply 110, an energy delivery device 120, an ultrasound navigation or localization system 130, a communications interface 140, a display 150, and a controller 160. The navigation system 130 utilizes reference catheters 132 having ultrasound transducers 131. The energy delivery device 120 includes an ablation catheter 122 with ultrasound transducers 121 and an electrode 127 for delivering energy 112 from the RF generator 111 of the power supply to the patient 170. Persons of ordinary skill in the art will appreciate that embodiments of a gating system can also be used with navigation of non-ablation catheters. For example, electrophysiology (EP) catheters, the gating system can be used to guide "mapping catheters". Thus, although some catheters may not support ablation and thermometry, they can include electrodes and navigation transducers so that their position can be tracked or navigated during RF ablation with another catheter. This specification, however, refers to ablation catheters for purposes of explanation, not limitation.

The controller 160 generates timing signals 162 for driving the RF generator 111 of the power supply 110, and timing signals 164 for driving the navigation system. If necessary, the interface 140, such as an optical interface, can format or configure the timing signals 164 into timing signals 165 that are suitable for driving the navigation system 130. The navigation system 130 establishes a reference system 137 using ultrasound energy or signals 133 (generally "signals") between transducers 131. The navigation system also obtains and processes data 124 related to ultrasound energy or signals (generally "signals") sent and received between ablation catheter transducers 121 of the energy delivery device 120 and reference catheter transducers 131.

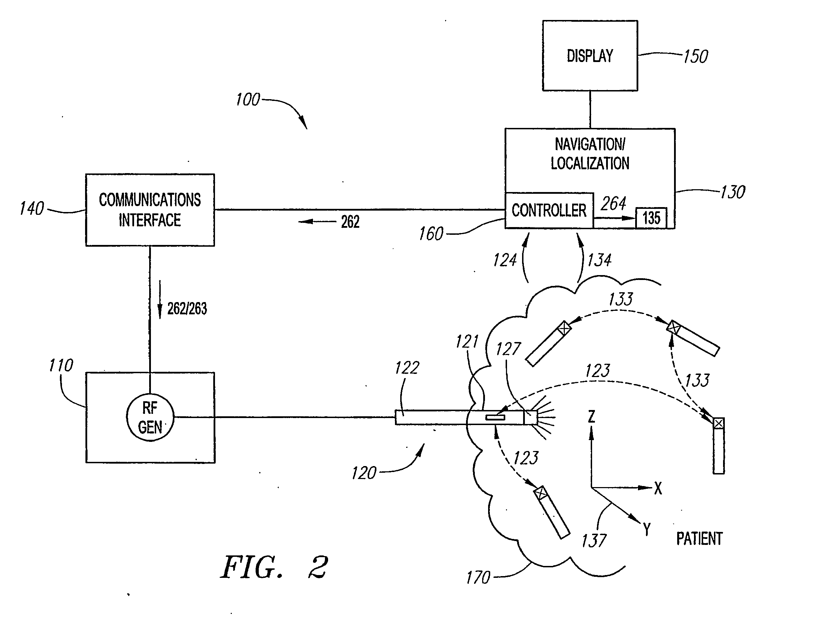

The embodiment shown in Figure 1 includes a controller 160 that is a component of the power supply 110. Other system configurations can also be utilized. For example, as shown in Figure 2, the controller 160 is a component of the navigation system 130. In the embodiment shown in Figure 2, the controller 160 generates timing signals 264 to drive the navigation system 130 and timing signals 262 to drive the RF generator 111. If necessary, the interface 140 can format or configure the timing signals 262 into timing signals 263 that are suitable for driving the RF generator 111.

Persons of ordinary skill in the art will recognize that various controller 160 configurations can be utilized. For example, the navigation system 130 can have exclusive control over timing signals, the power supply 110 can have exclusive control over timing signals, or the control can be distributed or shared between the power supply 110 and the navigation system 130. For purposes of explanation and illustration, but not limitation, this specification primarily refers to the configuration shown in Figure 1, in which the controller 160 is a component of the power supply 110.

The navigation or localization system 130 generates a 3-D reference coordinate system 137, grid or map using the ultrasound transducers 131 of the reference catheters 132. The reference catheters 132 are inserted through the body to a target area in the patient 170, such as the heart. The system 100 can be used in other regions of the body, but this specification describes applications involving the heart for purposes of explanation, not limitation.

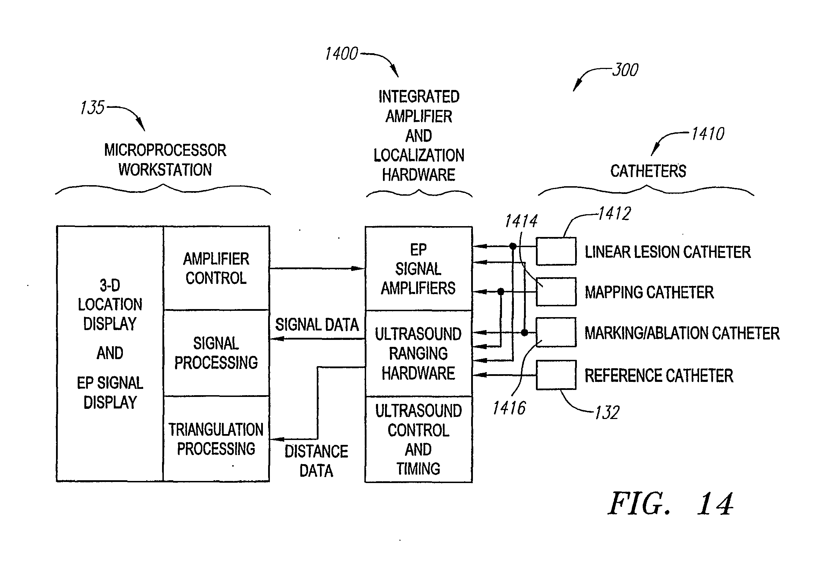

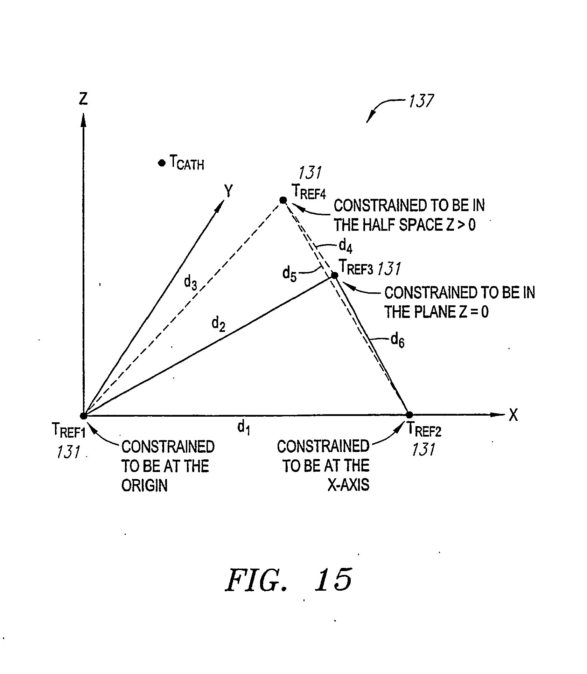

The transducers 131 carried by the reference catheters 132 send and receive signals (generally 133) to and from each other. A processor 135 acquires data (generally 134) related to the time it takes for the signals 133 to be transmitted and received between each of the reference catheter transducers 131. The processor 135 uses this data 134 to determine the distances between transducers 131 within the body using, for example, "time of flight" calculations. The processor 135 triangulates these distances to establish the positions of the reference transducers 132 relative to each other and to establish the 3-D coordinate system 137. The established coordinate system 137 can be represented graphically in 3-D on the graphics display 150 for use by a clinician or physician. The time of flight and triangulation determinations are explained in further detail with reference to Figures 14 and 15.

Once a 3-D coordinate system 137 is established, a physician can guide an energy delivery device, such as a mapping or ablation catheter 122, to the target area 170. The location of an ultrasound transducer 121 of the ablation catheter 122 can be determined in relation to the established coordinate system 137 with signals 123 that are transmitted between one or more transducers 121 of the ablation catheter 122 and one or more transducers 131 of the referenced catheters 132. The processor acquires data 124 related to the relative position of a transducer 121 relative to a transducer 131 to accurately determine the position of the catheter 123 within the reference grid 137 and the heart 170.

After an ablation catheter 122 is properly positioned, the RF generator 111 is activated to provide current 112 through an electrode 127 of the ablation catheter 120 to the target heart tissue 170.

Thus, the transducers 121 and 131 used in the navigation system 130 are different than typical ultrasound transducers of conventional imaging or visualization systems. For example, the transducers 121 and 131 of the ablation catheter 120 and the reference catheter 132 are used to establish a reference system and to determine positions with the reference system. Further, the transducers 121 and 131 are isotropic, i.e., they emit unfocused ultrasound energy in all directions so that the ultrasound energy can be detected by other transducers. In contrast, in conventional ultrasound imaging or visualization systems, ultrasound transducers typically emit focused, controlled ultrasound energy, which is directed to a particular target area. This focused energy is reflected, detected, and processed to produce an image of the target area.

Timing signals 162 and 164 activate the RF generator 111 and navigation system 130 at different times, so that the current 112 produced by the RF generator 111 does not significantly interfere or does not interfere with the transmission of ultrasound signals between various transducers, including the receiving and transmitting such signals. Thus the RF current 112 does not significantly interfere with ultrasound signals 123 that are transmitted between ultrasound transducers 121 and 131 of catheters 120 and 132, signals 133 transmitted between various ultrasound transducers 131 of reference catheters, and acquisition of the corresponding data 124 by the processor 135.

The phase and duty cycle of the timing signals 162 and 164 are programmed so that they "alternate" with respect to each other. Thus, the RF generator 111 and output 112 are active when the navigation system 130 and ultrasound signals 123 are inactive. Conversely, the ultrasound signals 123 are active when the RF generator 111 and output 112 are inactive.

As a result, the position of an ablation catheter 120 that includes an ultrasound transducer 121 can be monitored and adjusted, as necessary, before and during RF ablation since the position of the ultrasound transducer 121 of the catheter 120 is accurately monitored relative to the established 3-D reference coordinate system 137. The position of the ablation catheter 120 can be presented as a graphic rendering or representation on the display 150 for the clinician.

Persons of ordinary skill in the art will recognize that the same "interference" problems and gating or alternating technique can be used with other navigation signals, including electromagnetic (EM) navigation or localization systems. For example, high or maximum power RF energy can interfere with EM signals emitted by reference or ablation catheters. This specification, however, refers to ultrasound transducer signals for purposes of explanation, but not limitation.

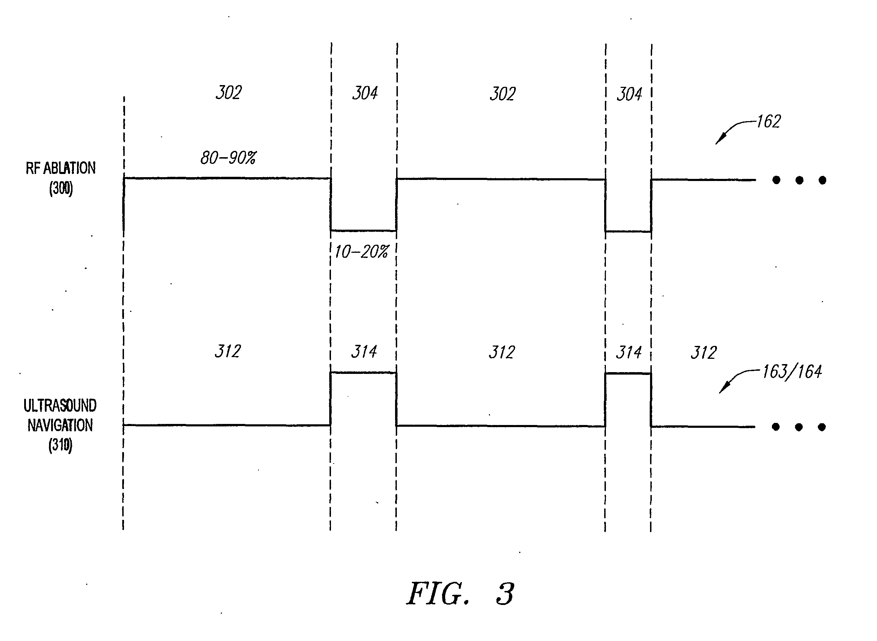

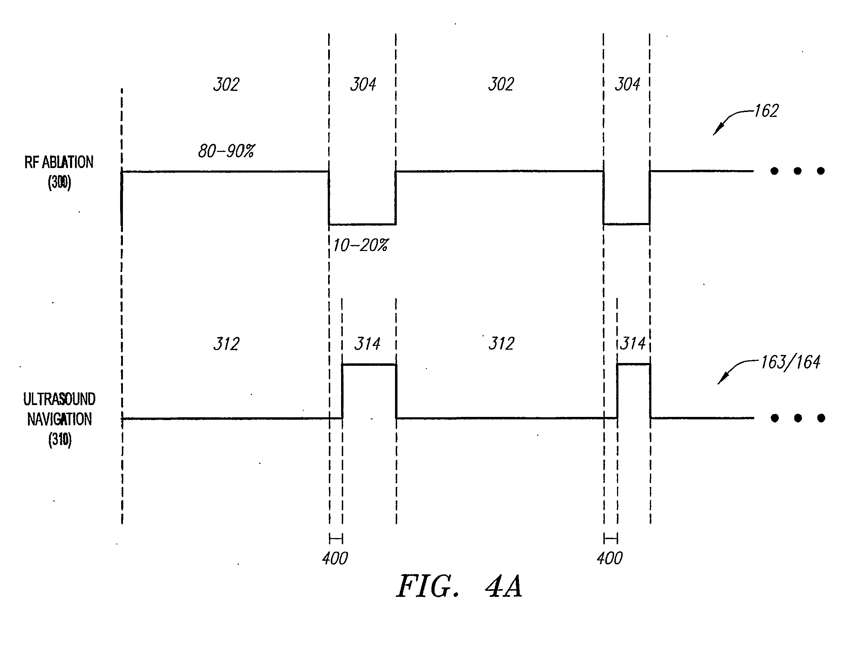

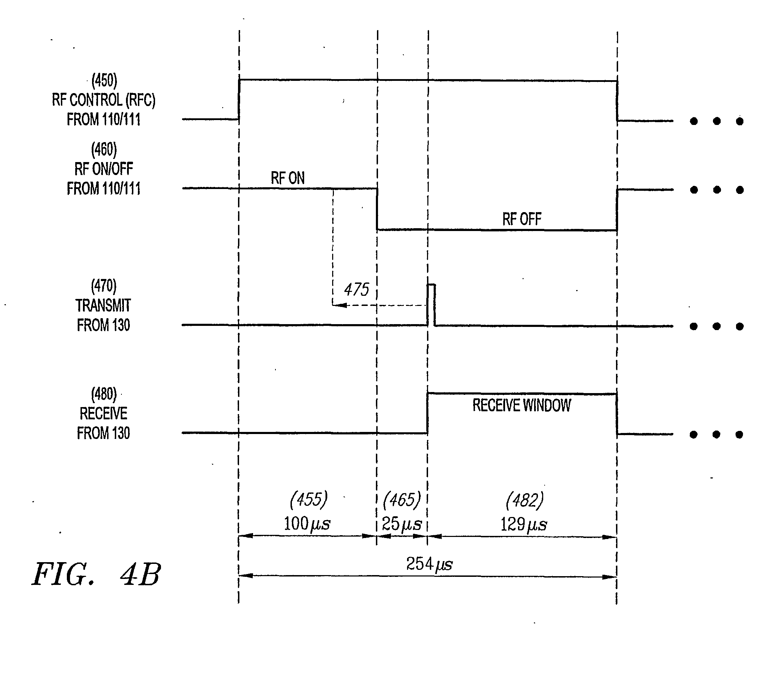

General timing principles are illustrated in Figures 3 and 4A, and a specific implementation is illustrated in Figure 4B. These signals and control of the timing can be implemented in hardware and/or software to drive the RF generator 111 and ultrasound transducers 121 and 131 so that the current produced by the RF generator does not interfere or does not significantly interfere with the transmitting and/or receiving of ultrasound signals by the navigation / localization system 130.

In one embodiment, as shown generally in Figure 3, the RF generator 111 is active to perform RF ablation 300 when the timing signal 162 to the RF generator 111 in the power supply 110 is high 302, whereas the RF generator 111 and RF ablation 300 are inactive when the timing signal 162 is low 304. In one embodiment, the RF generator 111 is active for a longer time relative to the ultrasound signals. For example, the control circuit 160 can be programmed to have a 80%-90% duty cycle. In other words, the RF generator 111 (and RF ablation 300) are active for about 80-90% of a time period and inactive for about 10-20% of the time period.

For example, for every 1 ms, the RF generator 111 and ablation 300 are active for about 800-900 microseconds, preferably about 825-875 microseconds, and inactive for about 100-200 microseconds, preferably for about 125-175 microseconds. Other heat treatment of the tissue, e.g., convective heating, may also continue during the off periods 304 since heat energy remaining in the tissue may still treat the tissue during the short time that the power supply RF generator 111 and RF ablation are de-activated. Similarly, ultrasound signals and acquisition and navigation 310 are inactive during periods 312 (when the RF generator 111 is on) and active during periods 314 (when the RF generator 111 is off).

The power over time or average or "RMS" power resulting from these duty cycles has been determined to provide sufficient current and power to effectively ablate tissue, while reducing or preventing tissue charring and blood coagulation. These duty cycles also allow a sufficient amount of ultrasound information 121 to be acquired so that the position of the ablation catheter 120 can be accurately determined and monitored before and during RF ablation. If variable ablation capabilities are necessary, the peak power level of the power supply 110 can be adjusted, as necessary, to provide more or less ablation power. For example, an operator can adjust the "average power" or "rms power" setting on the power supply 120, which results in the peak power being adjusted to provide the selected average or rms power output.

Persons of ordinary skill in the art will recognize that a beginning of an ultrasound cycle may not perfectly align with an end of a RF ablation cycle due to, for example, system or component tolerances. Thus, while Figure 3 illustrates substantially non-overlapping timing signals, in use, there may be a small degree of overlap that can be tolerated, while still effectively switching between RF ablation 300 and ultrasound navigation 310.

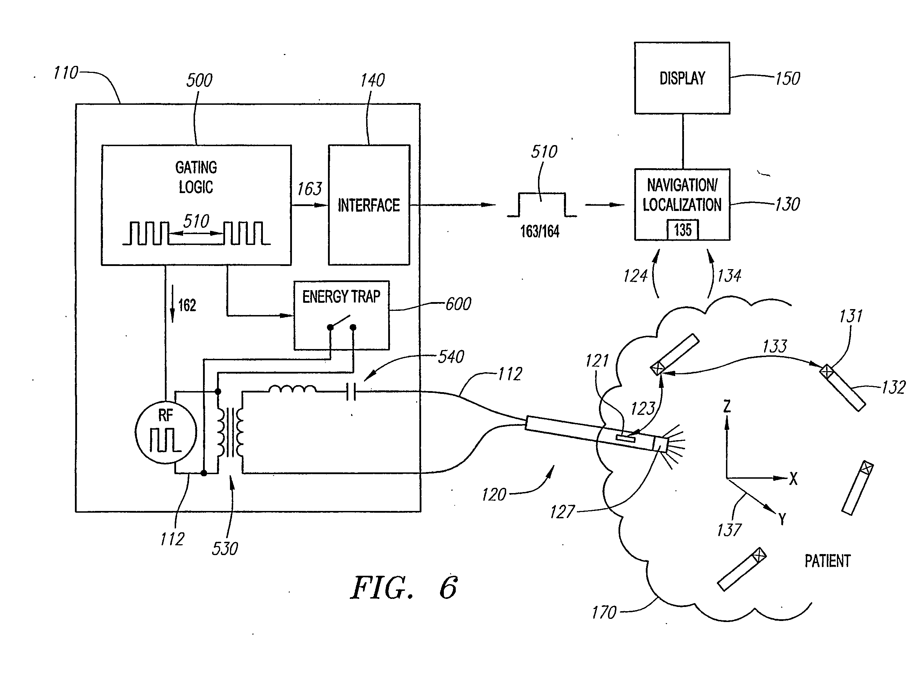

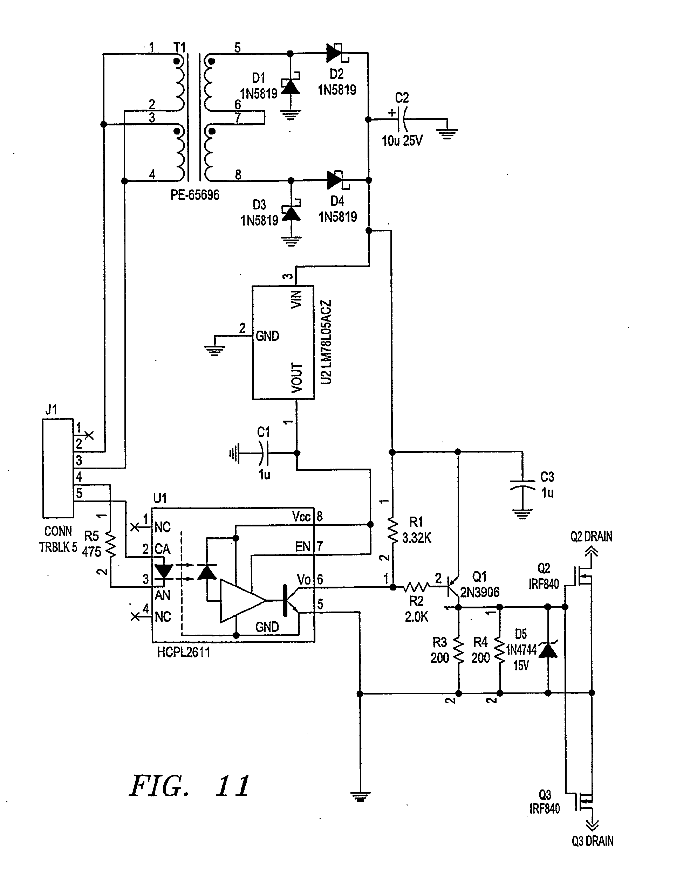

In an alternative embodiment, as shown generally in Figure 4A, the ultrasound navigation timing signals 164 may be delayed as necessary in order to compensate for residual RF signals and system and component tolerances. For example, as shown in Figure 4A, the controller 160 may introduce a delay 400, e.g., about 0 to about 100 microseconds, preferably about 25 microseconds, before activating signals between transducers and ultrasound acquisition. The delay 400 ensures that residual RF signals that exist in filter and magnetic components of the power supply 110 decay to a zero or substantially low level so that they do not interfere with the ultrasound signals 121 and acquisition 124. In an alternative embodiment, the system 100 includes an "energy trap" circuit that accelerates the decay of residual RF signals before beginning an ultrasound cycle, as discussed in further detail below with reference to Figures 6 and 11.

A more specific timing implementation of one embodiment is shown in Figure 4B. This embodiment provides flexibility to change the time that the navigation /localization system 130 activates a transducer to transmit ultrasound signals. This flexibility is particularly useful if ultrasound signals are transmitted for longer distances. For example, typical ultrasound transmission distances may be for about 200 mm, and longer distances, such as from about 150 mm to about 300 mm may result from receivers being placed on the skin of the patient. Accordingly, various embodiments are adaptable to systems that use internal ultrasound transmitters and receivers and a mixture of internal and external transmitters and receivers.

Referring to Figure 4B, four signals are utilized to control the timing of RF ablation and ultrasound transmissions: RF Control (RFC) 450, RF On/Off 460, Transmit 470 and Receive 480. The RFC signal 450 is provided from the power supply 110 or RF generator 111 and to the navigation / localization system 130. The RF On/Off signal 460 is the output of the power supply 110 or RF generator 111. As illustrated in Figure 4B, the RF On/Off signal 460 is an "active high" signal. In other words, the RF generator 111 is off when low and on when high. In practice, the RF On/Off signal 460 may be active low - the RF generator 111 is on when the signal 460 is low and off when the signal is high. The Transmit signal 470 is a signal generated by the navigation / localization system 130 to control when a transducer is activated to emit ultrasound signals. The Receive signal 480 is also generated by the navigation / localization system 130 to enable the receiver detection circuitry during a receive window 482.

In use, the transition of the RFC signal 450 from low to high serves to mark the beginning of a window 455 (100 microseconds in this example) before which the RF generator 111 is activated by the RF On/Off signal 460. Thus, as illustrated, the ultrasound navigation system 130 triggers the Transmit signal 470 shortly after the RF generator 111 is turned off - about 25 microseconds 465 after the RF generator 111 is de-activated in this example. Similarly, the ultrasound / navigation system 130 provides the Receive signal 480 to activate one or more transducers to enable receiver detection circuits during the receive window 482 of about 129 microseconds.

If longer transmission distances are utilized, the Transmit signal 470 can be triggered earlier. For example, the Transmit signal 470 can be triggered earlier, i.e., while the RF generator is still on, as shown by 475. In other words, the trigger can be activated during the 100 microsecond window 455 and while the RF generator is on. As a result, the ultrasound signals can be transmitted for a longer time, and thus, a longer distance, while being received during the window 482 and while the RF generator is not active.

Indeed, persons of ordinary skill in the art will appreciate that the previous exemplary durations of signals are merely representative of other durations that can be used with various embodiments and applications. Accordingly, the specific examples involving 25, 100, 125, 129 and 254 microsecond timing intervals are not to be considered to limit the various embodiments.

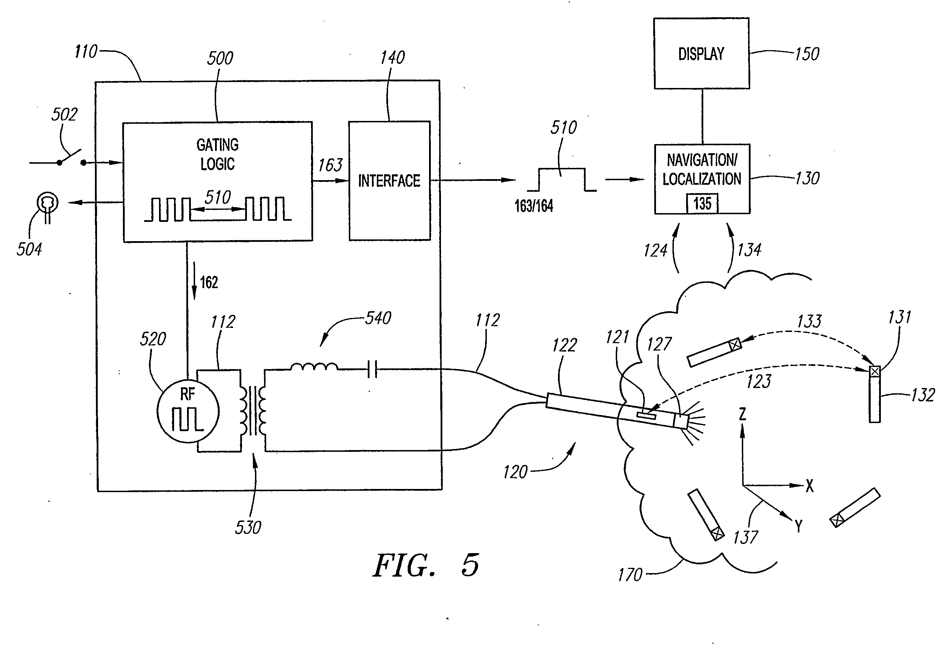

Figure 5 illustrates a gating system 100 in further detail. The illustrated embodiment includes the interface 140 within the power supply 110, however, the interface 140 can also be external to the power supply 110, as shown in Figure 1. In the illustrated embodiment, the system 100 includes a controller 160, such as a micro-controller, microprocessor, programmable logic device (PLD), discrete logic or other suitable logic device (generally, "gating logic" 500). Various logic languages can be used to program different logic devices, as necessary, including but not limited to Hardware Description Language (HDL) and VHSIC Hardware Description Language (VHDL).

The gating logic 500 is initially activated by a user via a switch 502. An indicator 504 informs the user that the gating logic 500 has been activated. The indicator 504 may be, for example, a visual indicator, such as a Light Emitting Diode (LED), or an audible indicator, such as a beep or a tone.

The gating logic 500 generates timing signals 162 (RF Clock) to drive the RF generator 111 and timing signals 164 (Gating Sync - Optical Out) to drive the navigation system 130. The timing signals 162 and 164 are synchronized so that during a gating interval 510, the RF Clock timing signal 162 is switched low to de-activate the RF generator 111, and the Gating Sync timing signal 164 is switched high to drive the ultrasound transducers 121 and 131 and ultrasound acquisition. As a result, ultrasound transmissions and detections of signals between transducers 121 and 131 can occur during the "quiet" gating period 510 without interference from ablation current 112. This data 124 can be acquired by the processor 135 to determine the location of the ablation catheter transducer 121 relative to the reference system 137.

The RF generator 111 can be various known switching generators that provide electrical current 112 having frequencies between about 200 kHz and about 800 kHz, preferably about 500 kHz, and up to 2 A of current and 170 V. The RF generator output 112 is Coupled by a transformer 530. A bandpass filter 540 (a series LC network) shapes the transformed output 112 to a 0-170 volt sinusoidal signal having a frequency of about 500 kHz for the ablation catheter 120.

Referring to Figure 6, an alternative embodiment of a gating system includes an "energy trap" or RF decay circuit 600, such as a solid state switch. The energy trap circuit 600 accelerates the decay of residual RF signals that exist in, for example, the bandpass filter 540 and magnetic components of the transformer 530, after the RF energy has been de-activated, i.e., at the beginning of each "gating" period 510 prior to ultrasound acquisition 124.

More particularly, during a gating interval 510, the gating logic 500 stops the RF clock 162 and closes the energy trap switch 600. As a result, RF energy within a bandpass filter 540 does not leak to the ablation electrode 127 output. The energy trap 600 provides a faster transition from an active or high RF signal to a zero or low level that does not interfere with ultrasound signals 121 and 131 and acquisition 124. If necessary, the gating logic 500 can introduce a small delay, e.g., about one microsecond, after the RF generator 111 is switched off and before closing the energy trap switch 600, to ensure that the RF generator 111 is turned off.

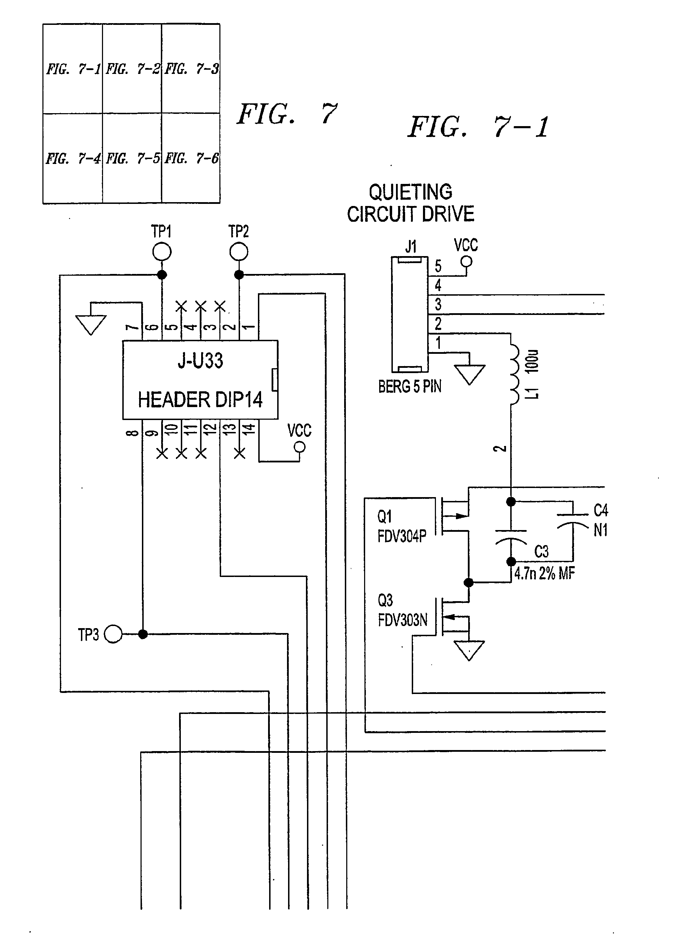

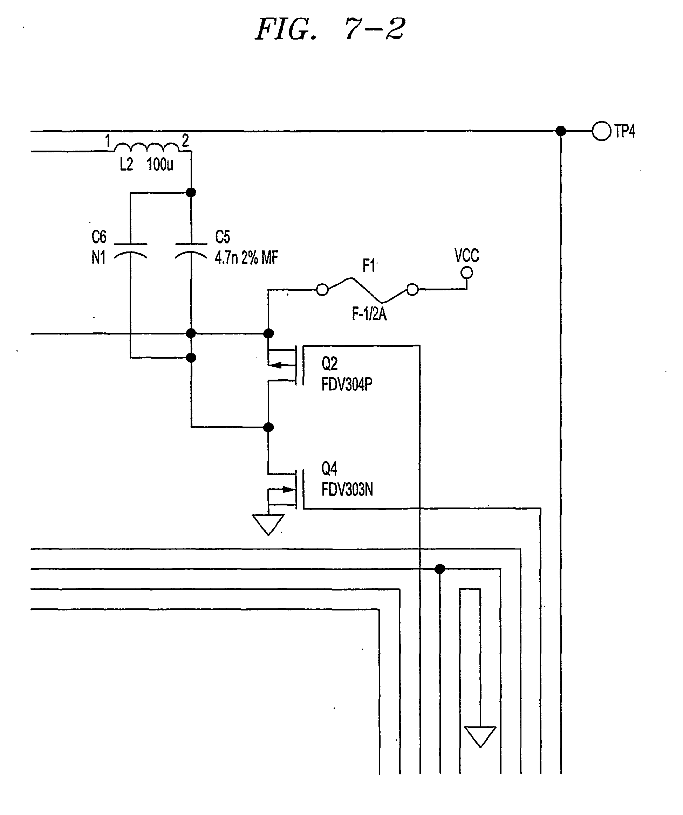

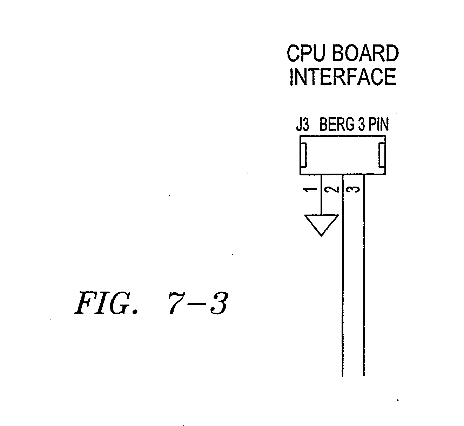

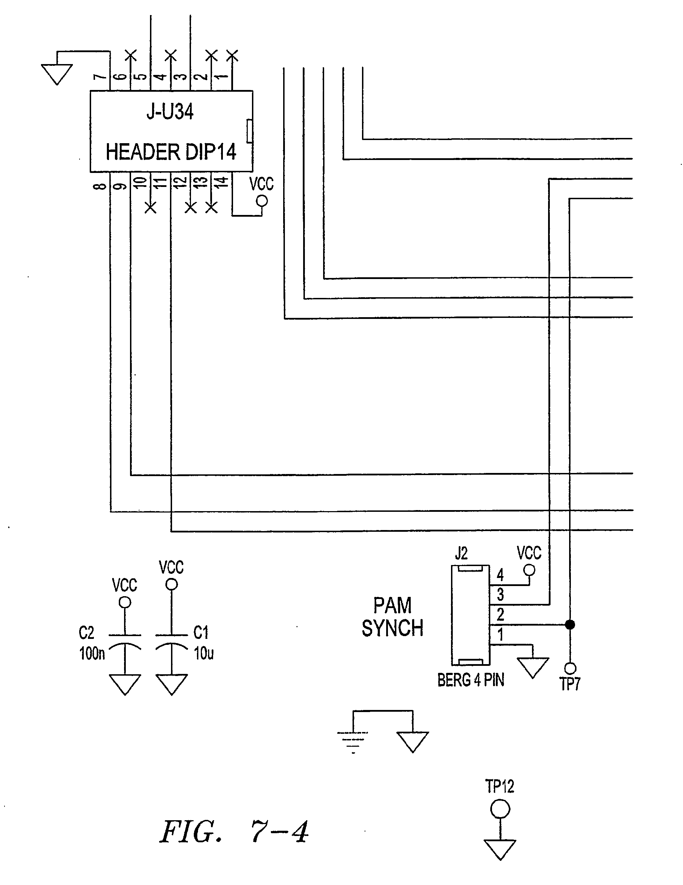

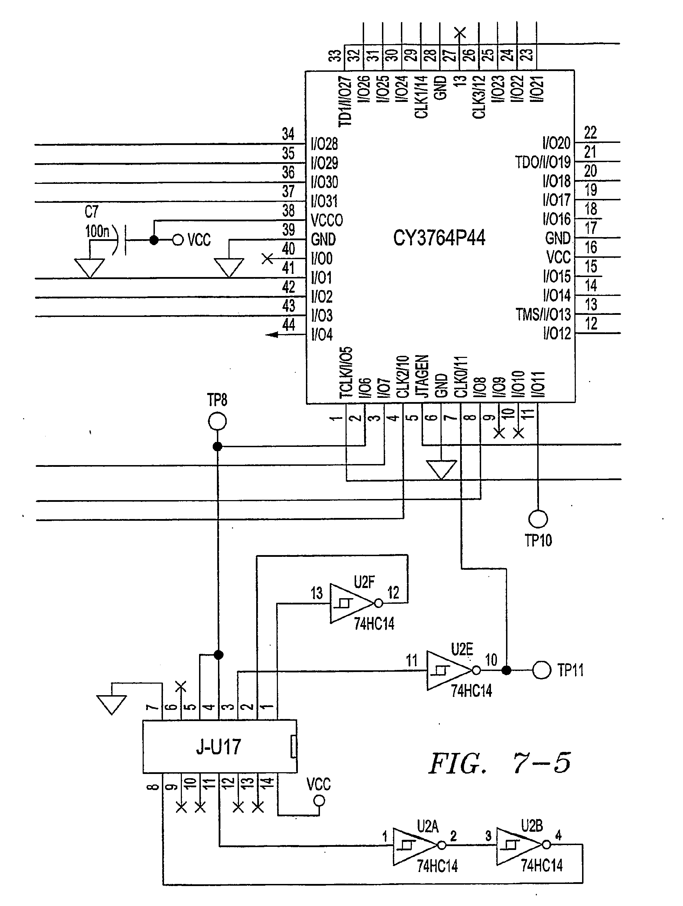

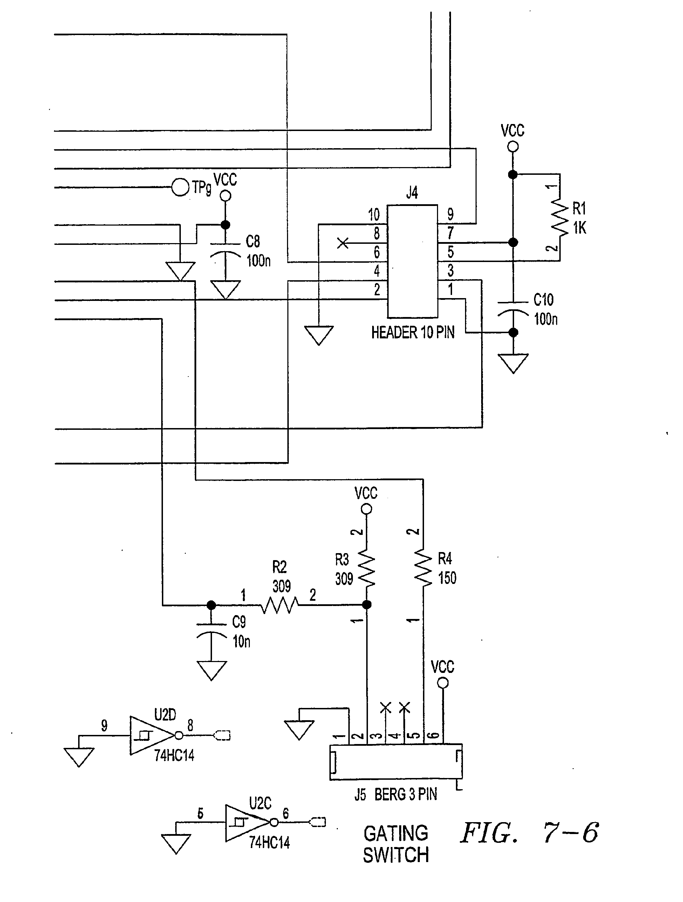

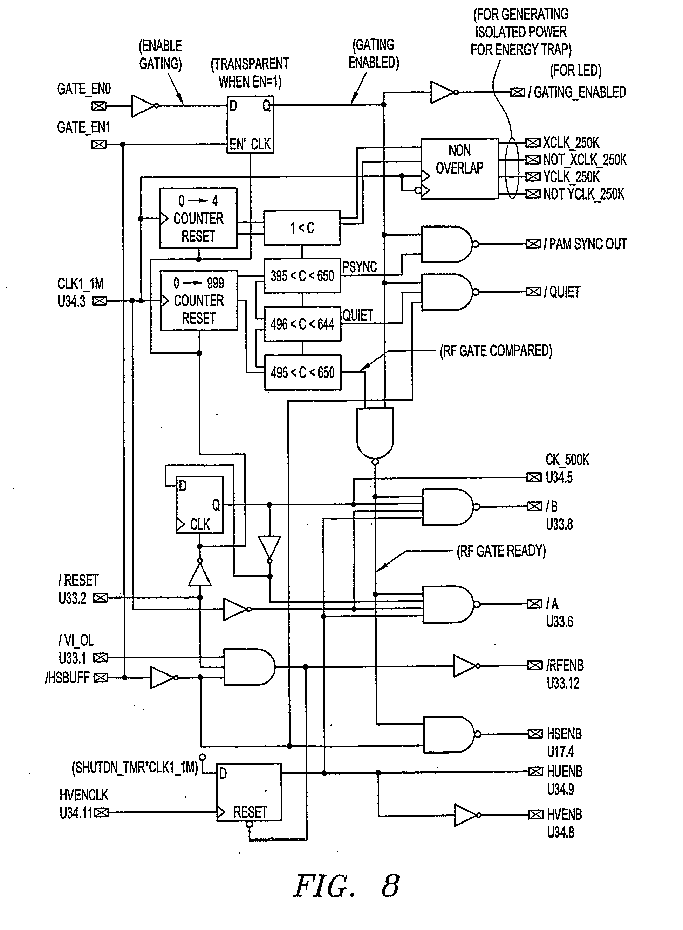

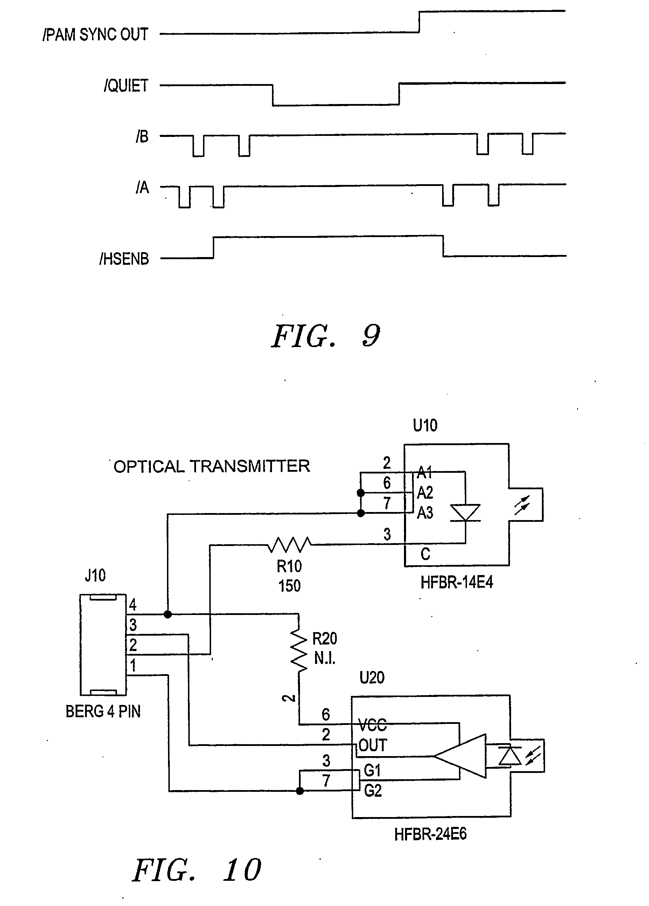

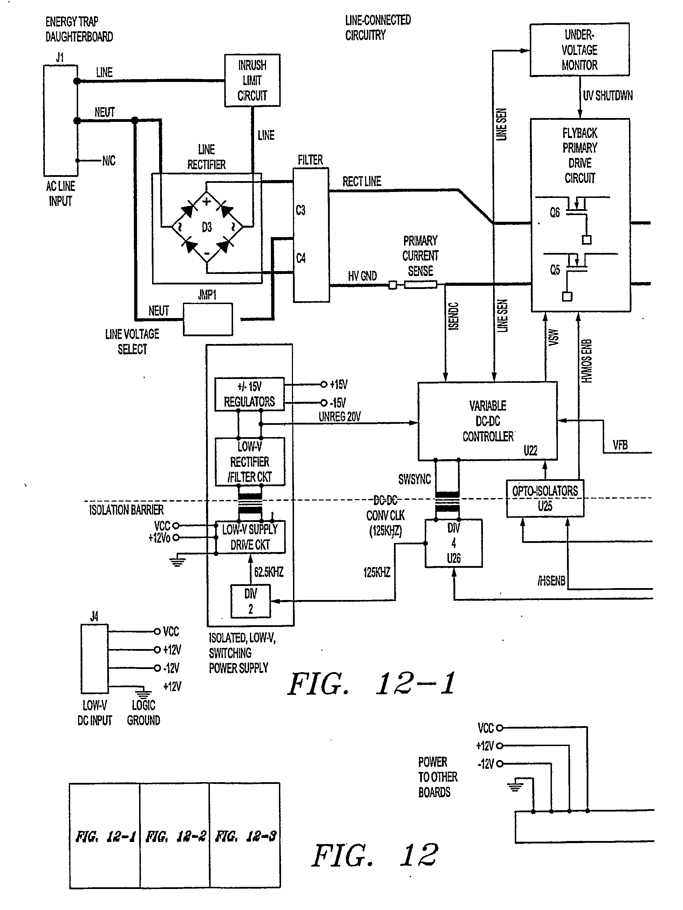

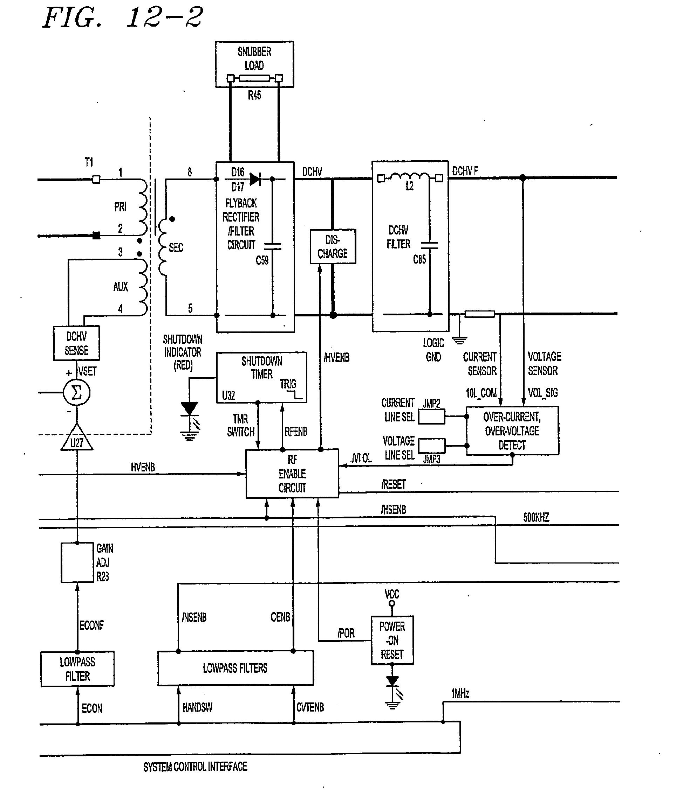

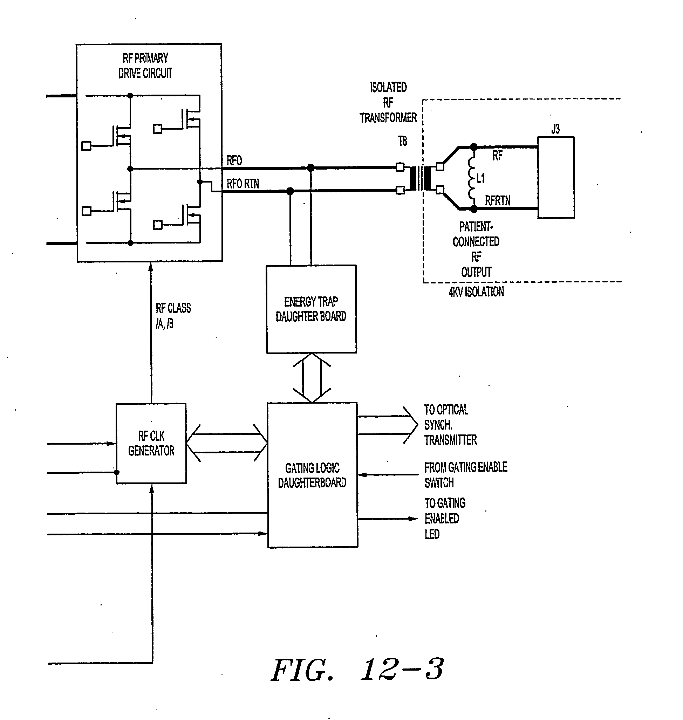

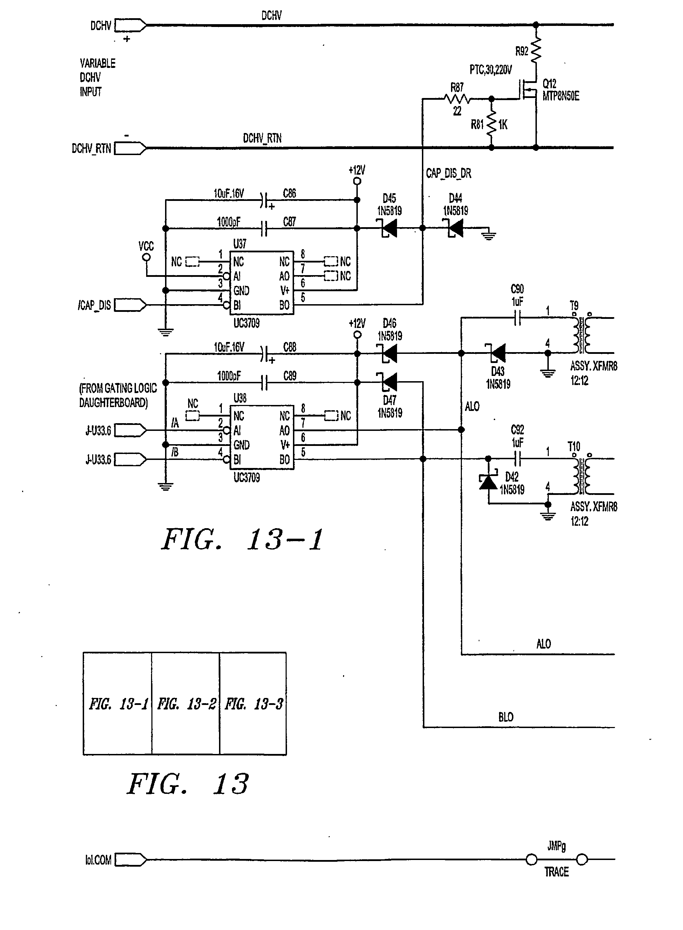

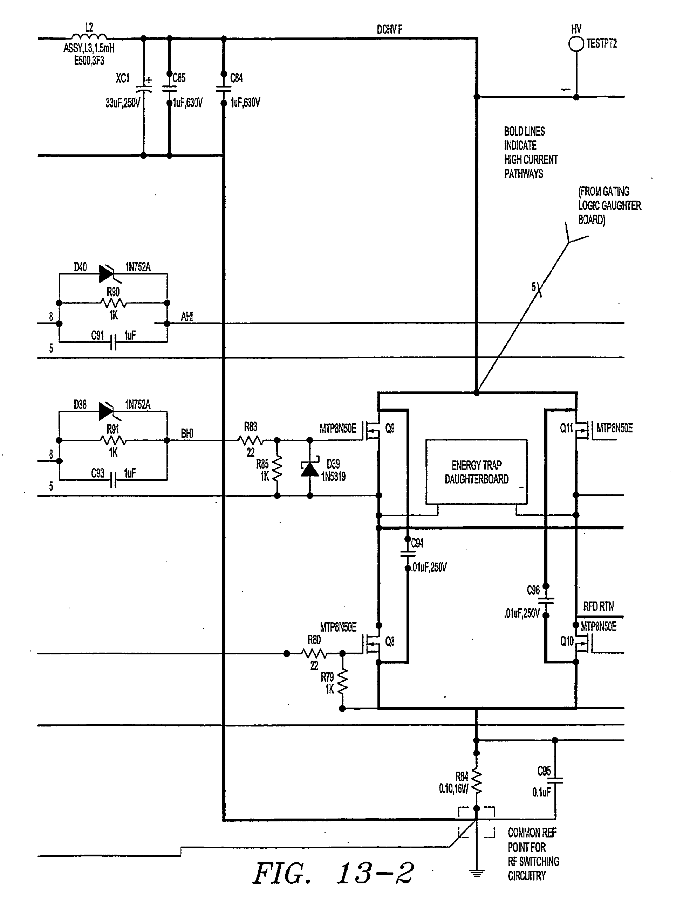

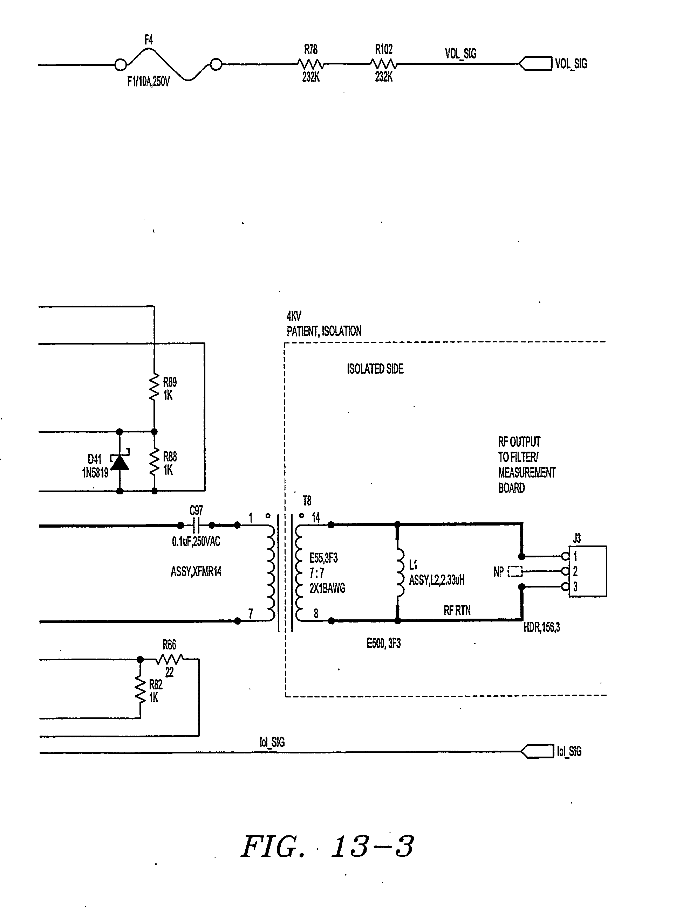

Figures 7-13 illustrate circuit, logic and timing diagrams that correspond to portions of components of the system block diagrams of Figures 1, 2, 5 and 6. These particular circuit diagrams are configured for use with existing ultrasound navigation products, including PAM model no. 8200 and PAMi part no. 09-1685-000 manufactured by Boston Scientific Corporation 2710 Orchard Parkway, San Jose, California 95134, and generally described in U.S. Patent No. 6,490,474. Accordingly, persons of ordinary skill in the art will recognize that embodiments of a gating system can include different circuit components and can be configured for other navigation systems and applications as necessary. Thus, the circuit and logic schematics shown in Figures 7-13 are illustrative of various other circuit designs that can be utilized to implement switching between ultrasound navigation 300 and RF ablation 310. The manner in which the ultrasound navigation system 130 components operate to generate the 3-D reference coordinate system using time of flight and triangulation determines is now described in further detail.

Referring to Figure 14, as previously discussed generally, the navigation or localization system 300 and procedure utilize ultrasound transducers 131 of reference catheters 132 to establish a 3-D reference coordinate system 137 within a patient's heart 170. Each reference catheter 132 includes a plurality of ultrasound transducers 131 - preferably at least four such transducers 131. The reference catheter transducers 131 function as ultrasound receivers by converting acoustic pressure to voltage, and as ultrasound transmitters by converting voltage to acoustic pressure.

Using known techniques and calculations, the distance between each reference catheter transducer 131 and other ones of the reference catheter transducers 131 may be computed by measuring the time for an ultrasound pulse to travel from a transmitting transducer 131 to each receiving transducer 131. These distance measurements are preferably carried out in parallel so that when an ultrasound pulse is emitted by a reference catheter transducer 131, the system simultaneously measures the respective times it takes for the pulse to reach each of the other transducers being used in the system.

These determinations are made based on the velocity of an acoustic signal in the heart being approximately 1570-1580 mm/msec, with small variations caused by, for example, blood and tissue. The time for an acoustic pulse to travel from one transducer 131 to another may thus be converted to the distance between the transducers 131 by multiplying the time of flight by the velocity of an acoustic pulse in the heart (i.e. by 1570-1580 mm/msec).

Time of flight principals, in combination with the geometric principal of triangulation, establish the 3-D coordinate system 137 using the processor 135, amplifier and localization hardware 1400 and catheters (generally 1410). One or more of the reference catheters 132 is introduced into the heart 170 or the surrounding vasculature (or other areas such as the esophagus) and is left in place for the duration of the procedure. Once reference catheter(s) 132 are positioned within or near a patient's heart 170, the system first measures the distances between each of the reference catheter transducers 131 using "time of flight" principles. The processor 135 uses these distances to establish the relative positions of the reference transducers 131 and to establish a 3-D coordinate system 137.

For example, referring to Figure 15, a 3-D coordinate system 137 is established by using a reference catheter 132 that includes at least four reference transducers 131, designated T1-4. These transducers T1-T4 define a 3-D coordinate system 137 as follows: T1 through T3 define the plane P at z=0; one reference transducer T1 defines the origin of the coordinate system; a line between T3 and T2 defines the x-axis of the system; and T3 lies in the plane z=0. The fourth reference transducer, T4, lies on one side of the plane P, at z>0.

The reference catheter 132 preferably includes at least four such transducers 131 so that a 3-D coordinate system 137 can be established using a single reference catheter 132. If desired, the reference catheter 132 may have more transducers 131 or it may have fewer transducers 131 if more than one reference catheter 132 is to be used to establish the three-dimensional coordinate system 137.

Using more than four reference transducers 131 is advantageous in that it adds redundancy to the system and thus enhances the accuracy of the system. When more than four reference transducers 131 are used, the problem of determining the location of catheter transducers 131 is over determined. The additional redundancy may provide greater accuracy if the measured distances between the reference transducers 131 and catheter transducers 121 are noisy. The overdetermined problem can be solved using multi-dimensional scaling as described in "Use of Sonomicrometry and Multidimensional Scaling to Determine 3D Coordinates of Multiple Cardiac Locations: feasibility and implementation", Ratciffle et. al, IEEE Transactions Biomedical Engineering, Vol. 42, no. 6, June 1995.

The coordinates of the reference transducers 131 can be computed using the law of cosines. See, for example, Advanced Mathematics, A preparation for calculus, 2nd Ed., Coxford, A. F., Payne J. N., Harcort Brace Jovanovich, New York, 1978, p. 160. Each reference transducer 131 must be capable of both receiving and transmitting ultrasound pulses, and is separately made to emit acoustic pulses that are received by each of the other reference transducers 131. The distances d1 through d6 shown in FIG. 15 are calculated using the respective times it takes for an acoustic pulse to travel between each pair of the reference transducers 131. These distances are triangulated to establish the positions of the reference transducers 131 relative to each other, and therefore to establish a 3-D coordinate system 137.

Referring again to Figure 14, once a 3-D coordinate system 137 is established, the location of an additional transducer 121 of a medical device 120, such as a mapping or an ablation catheter (generally 1410 in Figure 14) placed near or within the heart can be calculated within the 3-D coordinate system 137 as follows. First, using the "time of flight" method, the distances between each of the reference transducers 131 T1 through T4 and the additional catheter transducer 121 (designated TCATH in FIG. 15) are established, in parallel. These distances are preferably also performed in parallel with the distance measurements that are made to establish the coordinate system 137. Next, using basic algebra and the law of cosines (see, e.g., the Advanced Mathematics text cited above), the coordinates of TCATH 121 relative to the reference transducers 131 are calculated using the measured distances from T1 through T4 to TCATH 121, referred to as triangulation.

The locations of all or portions of the reference catheters 132 may be displayed as well. The system is preferably programmed to extrapolate catheter position from the coordinates of the transducer locations based on models of the various catheters pre-programmed into the system, and to display each catheter's position,and orientation on a graphical user display (see display 150 in FIG. 1). The locations of all or portions of the additional catheters 120 and transducers 121 (such as, for example, their distal tips, their electrodes or ablation sections, if any, or other sections which may be of interest) are displayed. The reference catheter(s) 132 thereby establish an internal coordinate system 137 by which the relative positions of EP catheter transducers 121 in the heart may be calculated using triangulation and shown in real-time on a three dimensional display 150.

A location of a distal tip of a catheter 122 (and thus the location of the anatomical site) can also be extrapolated from the transducer 121 location using a pre-programmed model of the catheter 122. For example, the processor 135 can graphically reconstruct a representation of a heart chamber based on anatomical points acquired by the mapping / ablation catheter 122. More specifically, the processor 135 deforms a spherical surface model to the anatomical points as each is acquired within the heart. These constructed representations or renderings can be more accurate and useful than images generated by known ultrasound visualization systems, which typically generate images of a heart using conventional ultrasonic generation, reflection and detection, and image formation techniques. Additional details regarding a navigation system can be found in U.S. Patent No. 6,490,474.

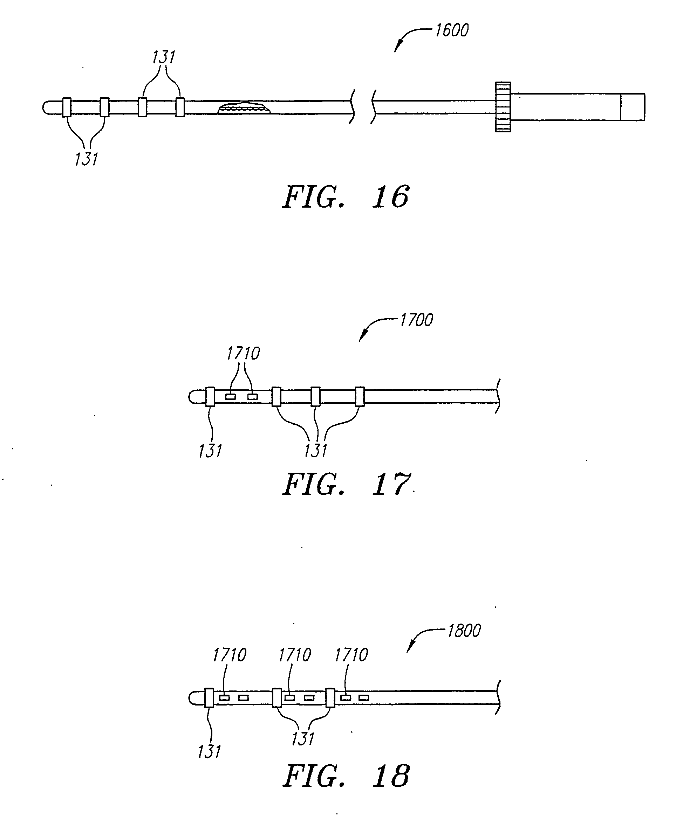

Figures 16-18 illustrate exemplary catheters. Referring to Figure 16, one exemplary reference catheter 1600 that can be used in an ultrasound navigation system 300 is an elongate catheter having a plurality of ultrasound transducers 131 positioned at its distal end. The transducers 131 are piezoelectric transducers capable of transmitting and receiving ultrasound signals. The reference catheter 1600 can be integrated with typical EP catheters by providing the ultrasound transducers described above. This allows the system to utilize the localization and navigation functions using catheters which are already needed for the EP procedure. Thus, use of the system does not require the physician to use more catheters than would be used had the EP procedure been carried out without the localization function.

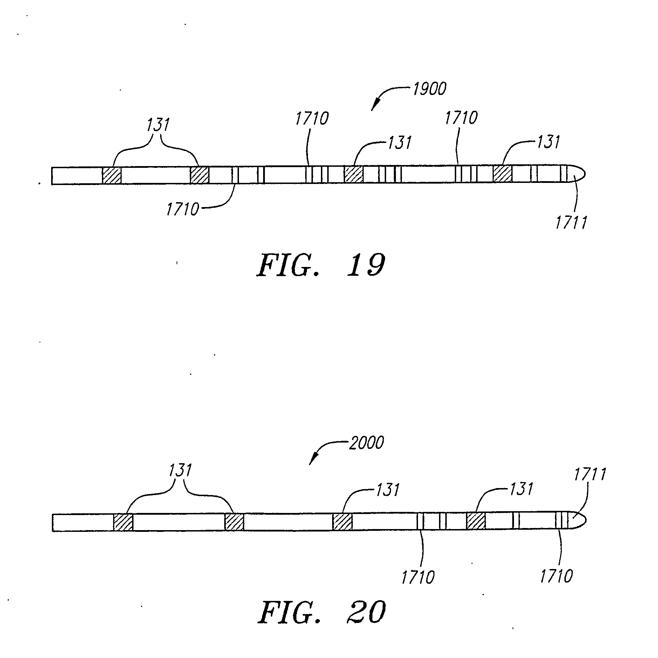

Another exemplary catheter is shown in FIG. 17. The reference catheter 1700 may be an RV apex catheter having a distal pair of EP electrodes 1710, an ultrasound transducer 131 at the distal tip, and additional ultrasound transducers 131 proximally of the distal tip. A further alternative catheter is a coronary sinus reference catheter 1800 (FIG. 18) that includes at least three bipole pairs of EP electrodes 1710 distributed over the section of the catheter that is positioned in the coronary sinus, and having at least three ultrasound transducers also distributed over the section of the catheter that is in the coronary sinus. Reference catheters that do utilize distal electrodes 1711, can also be utilized, such as the reference catheters shown in Figures 19 and 20.

The optimal operating frequency for the navigation system is determined by considering the resonant frequencies of the ultrasound transducers 121 and 131. It has been found that, given the dimensions and thus the resonances of the transducers being used in the system, the transducers are most preferably operated at a frequency of approximately 0.5 MHz, which can be the transducer resonance in the length mode. The transducers 121 and 131 are isotropic and have a beam width of approximately 114 degrees, where the beam width is defined as the angle over which the signal amplitude does not drop below 6 dB from the peak amplitude. If desired, a diverging lens in the form of a spherical bead of epoxy or other material may be formed over the ceramic cylinder to make the signal strength more uniform over the beam width.

A location of a distal tip of a catheter 122 (and thus the location of the anatomical site) can also be extrapolated from the transducer 121 location using a pre-programmed model of the catheter 122. The processor 135 can graphically reconstruct a representation of a heart chamber 170 based on anatomical points acquired by the mapping / ablation catheter 122. More specifically, the processor 135 deforms a spherical surface model to the anatomical points as each is acquired within the heart. These constructed representations or renderings can be more accurate and useful than images generated by known ultrasound visualization systems, which typically generate images of a heart using conventional ultrasonic generation, reflection and detection, and image formation techniques.

EP data can be acquired by positioning a mapping /ablation catheter 122 within an appropriate mapping location and activating the system to record EP data or activation points, which are sensed by pertinent mapping electrodes. Mapping electrode coordinates can be approximated and associated within the acquired EP data. If desired, an isochronal map (i.e., a color-coded image of activation time for underlying cardiac tissue) can be generated from the EP data and displayed in a 3-D context by superimposing the EP data over a graphical reconstruction of a heart chamber.

Alternatively, the physician can display a discrete map of activation points, in which case, the reconstructed heart chamber will not be displayed. Once the EP data is acquired and mapped onto the heart model surface, the mapping / ablation catheter can be steered to target sites identified by the EP data, and then operated to therapeutically ablate tissue at these sites.

In addition to monitoring the position of mapping or ablation catheters in the heart, impedance and temperature can be monitored during ablation. The system can be operated so that a power level or impedance level can alter the peak power of the power supply 110. For example, if the RF energy begins to char the heart tissue or if blood coagulates, the impedance and power wattage may increase, thereby signaling to the clinician that the power level should be reduced or the catheter can be re-positioned.

U.S. Patent No. 6,490,474 includes additional information relating to the 3-D reference coordinate system, time of flight, triangulation, reference catheters, and exemplary mapping and ablation catheter configurations 1500.

Persons of ordinary skill in the art will recognize that illustrated embodiments can be utilized to treat body tissues other than heart tissues. Further, other circuits and system configurations can be utilized as needed, as the illustrated circuit diagrams are provided to illustrate exemplary circuit components. Additionally, although this specification describes embodiments primarily with reference to reducing or eliminating interference with ultrasound signals, the same or similar principles and components can be used with electromagnetic signals. Further control over the timing signals can be exclusive or shared, and other duty cycles and delays can be utilized as necessary for different applications. Accordingly, the gating system is not limited to the particular exemplary embodiments described and illustrated, but modifications, alterations, and substitutions can be made to the described embodiments without departing from the accompanying claims.

## Claims

1. A system for positioning a medical device and ablating a tissue within a body comprising:
a power supply (110) for generating a current that is suitable for ablating the tissue and providing the current to an energy delivery device (120) the energy delivery device (120) configured to provide the current to the tissue to ablate the tissue;
a navigation system (130) for establishing a three-dimensional reference coordinate system and determining a position of the energy delivery device (120) within the reference coordinate system; **characterised in**
a controller (160) for switching between activating the power supply (110) and the navigation system (130) in accordance with timing signals (162,164) having duty cycles for the respective power supply (110) and navigation system (130) so that the navigation system (130) operates without significant interference from the power supply current during ablation of the tissue.

2. The system of claim 1, the power supply (110) including a radio-frequency generator.

3. The system of claim 1, the navigation system (130) being an ultrasound navigation system.

4. The system of claim 1, the timing signals (162,164) for the respective power supply (110) and navigation system (130) alternating with respect to each other.

5. The system of claim 1, the timing signals (162) for the power supply (110) having a duty cycle of about 80%-90%.

6. The system of claim 5, the timing signals (164) for the navigation system (130) having a duty cycle of about 10%-20%.

7. The system of claim 1, the duty cycles being programmed in the controller (160).

8. The system of claim 1, the duty cycles being fixed.

9. The system of claim 1, the controller (160) further comprising a circuit for accelerating a decay of the current provided by the power supply (110) before activating the navigation system (130).

10. The system of claim 1, the energy delivery device (120) comprising an ablation catheter.

## Patentansprüche

1. System zum Positionieren einer medizinischen Vorrichtung und zum Abtragen eines Gewebes innerhalb eines Körpers, welches aufweist:
eine Stromversorgung (110) zum Erzeugen eines Stroms, der geeignet ist zum Abtragen des Gewebes, und zum Liefern des Stroms zu einer Energiezuführungsvorrichtung (120), welche Energiezuführungsvorrichtung (120) ausgebildet ist zum Liefern des Stroms zu dem Gewebe, um das Gewebe abzutragen;
ein Navigationssystem (130) zum Errichten eines dreidimensionalen Bezugskoordinatensystems und
zum Bestimmen einer Position der Energiezuführungsvorrichtung (120) innerhalb des Bezugskoordinatensystems; **gekennzeichnet durch** eine Steuervorrichtung (160) zum Schalten zwischen Aktivieren der Stromversorgung (110) und
des Navigationssystems (130) gemäß Taktsignalen (162, 164) mit Tastverhältnissen jeweils für die Stromversorgung (110) und das Navigationssystem (130), so dass das Navigationssystem (130) ohne bemerkenswerte Interferenz **durch** den Versorgungsstrom während des Abtragens des Gewebes arbeitet.

2. System nach Anspruch 1, bei dem die Stromversorgung (110) einen Hochfrequenzgenerator enthält.

3. System nach Anspruch 1, bei dem das Navigationssystem (130) ein Ultraschall-Navigationssystem ist.

4. System nach Anspruch 1, bei dem die Taktsignale (162, 164) jeweils für die Stromversorgung (110) und das Navigationssystem (130) mit Bezug auf einander abwechseln.

5. System nach Anspruch 1, bei dem die Taktsignale (162) für die Stromversorgung (110) ein Tastverhältnis von etwa 80% bis 90% haben.

6. System nach Anspruch 5, bei dem die Taktsignale (164) für das Navigationssystem (130) ein Tastverhältnis von etwa 10% bis 20% haben.

7. System nach Anspruch 1, bei dem die Tastverhältnisse in der Steuervorrichtung (160) programmiert sind.

8. System nach Anspruch 1, bei dem die Tastverhältnisse festgelegt sind.

9. System nach Anspruch 1, bei dem die Steuervorrichtung (160) weiterhin eine Schaltung zum Beschleunigen einer Abnahme des von der Stromversorgung (110) gelieferten Stroms vor der Aktivierung des Navigationssystems (130) aufweist.

10. System nach Anspruch 1, bei dem die Energiezuführungsvorrichtung (120) einen Ablationskatheter aufweist.

## Revendications

1. Système pour positionner un dispositif médical et pour effectuer l'ablation d'un tissu dans un corps, comprenant :
une alimentation (110) pour générer un courant qui est approprié pour effectuer l'ablation du tissu et pour fournir le courant à un dispositif de délivrance d'énergie (120), le dispositif de délivrance d'énergie (120) étant configuré pour appliquer le courant au tissu afin d'effectuer l'ablation du tissu ;
un système de navigation (130) pour établir un système de coordonnées de référence tridimensionnelle et pour déterminer une position du dispositif de délivrance d'énergie (120) dans le système de coordonnées de référence ;
**caractérisé par**
un contrôleur (160) pour effectuer une commutation entre l'activation de l'alimentation (110) et du système de navigation (130) conformément à des signaux de cadencement (162, 164) qui présentent des rapports cycliques pour respectivement l'alimentation (110) et le système de navigation (130), de telle sorte que le système de navigation (130) puisse fonctionner sans provoquer une interférence significative avec le courant d'alimentation pendant l'ablation du tissu.

2. Système selon la revendication 1, l'alimentation (110) incluant un générateur radio-fréquence.

3. Système selon la revendication 1, le système de navigation (130) étant un système de navigation par ultrasons.

4. Système selon la revendication 1, les signaux de cadencement (162, 164) pour respectivement l'alimentation (110) et le système de navigation (130) s'alternant l'un par rapport à l'autre.

5. Système selon la revendication 1, les signaux de cadencement (162) pour l'alimentation (110) ayant un rapport cyclique d'environ 80%-90%.

6. Système selon la revendication 5, les signaux de cadencement (164) pour le système de navigation (130) ayant un rapport cyclique d'environ 10%-20%.

7. Système selon la revendication 1, les rapports cycliques étant programmés dans le contrôleur (160).

8. Système selon la revendication 1, les rapports cycliques étant fixes.

9. Système selon la revendication 1, le contrôleur (160) comprenant en outre un circuit pour accélérer une décroissance du courant fourni par l'alimentation (110) avant l'activation du système de navigation (130).

10. Système selon la revendication 1, le dispositif de délivrance d'énergie (120) comprenant un cathéter d'ablation.
